Europäisches Patentamt

**European Patent Office**

Office européen des brevets

(19)

(11) Publication number: **0 152 305 B1**

# EUROPEAN PATENT SPECIFICATION

(12)

(45) Date of publication of patent specification: **11.12.91**   (51) Int. Cl.⁵: **G01N 33/542**, G01N 33/563, G01N 33/577, G01N 33/535

(21) Application number: **85300991.8**

(22) Date of filing: **14.02.85**

(54) Method of measuring biological ligand by utilising amylase.

(30) Priority: **16.02.84 JP 27709/84**
**16.02.84 JP 27710/84**

(43) Date of publication of application:
**21.08.85 Bulletin 85/34**

(45) Publication of the grant of the patent:
**11.12.91 Bulletin 91/50**

(84) Designated Contracting States:
**CH DE FR GB IT LI NL**

(56) References cited:
**EP-A- 0 084 807**      **EP-A- 0 095 089**
**EP-A- 0 119 767**      **EP-A- 0 144 176**
**WO-A-82/01378**       **US-A- 3 935 074**

(73) Proprietor: **FUJIREBIO KABUSHIKI KAISHA also trading as FUJIREBIO INC.**
**6-7, Shimoochiai 4-chome Shinjuku-ku Tokyo 161(JP)**

(72) Inventor: **Ashihara, Yoshihiro**
**2-402, Fuchudanchi 28-1, Harumicho, 1-chome**
**Fuchu-shi Tokyo(JP)**
Inventor: **Kasahara, Yasushi**
**310, 4-4, Nagayama 3-chome ·**
**Tama-shi Tokyo(JP)**

(74) Representative: **Silverman, Warren et al**
**HASELTINE LAKE & CO. Hazlitt House 28 Southampton Buildings Chancery Lane London WC2A 1AT(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a method of measuring a biological ligand, for example, medicinal substances and trace components derived from various diseases which may be found in a humoral fluid such as blood or urine.

The measurement of the concentration in the blood of a drug administered to a patient, for example digoxin or theophylline, is important to the appropriate treatment, and the detection of trace materials derived from various diseases such as cancer which are to be found in the blood of an examinee is important in early diagnosis of the diseases.

A humoral fluid such as blood contains a variety of components, and some of them possess similar molecular weights or physiological properties. Hence the measurement of such components requires high specificity and high sensitivity. Furthermore, in order that it should be readily usable in an inspection method for diagnosis of a disease, the measuring procedure should be simple.

Various methods have been developed for detecting these trace components in blood and of these enzyme immunoassay has become widely employed because of its superior sensitivity and specificity and its capacity for use in the rapid treatment of a large number of samples. However, the sensitivity is not sufficient with conventional enzyme immunoassay and it is not easy to obtain an exact measurement of the concentration of a particular component because of the complicated washing procedures and the transferring of tubes which are involved.

The present inventors have investigated various enzyme immunoassays using various combinations of a ligand or an antibody and an enzyme or a material having an enzyme inhibitory activity as follows.

There is described in Japanese published patent application No.142466/1984 a method of measuring a biological ligand which comprises, contacting a ligand (1) to be measured and a combination of a ligand (2) having an antigenic determinant common to one of the antigenic determinant(s) of the above ligand (1) and biotin or a derivative thereof capable of reacting with avidin or streptoavidin as biotinyl enzyme inhibitor, with an antibody capable of reacting with the above common antigenic determinant in an aqueous solution, contacting the above combination with a biotinyl enzyme inhibitor such as avidin, streptoavidin or one of their derivatives capable of reacting with biotin, and measuring the residual biotinyl enzyme activity.

In the method of EP-A-0119767 for measuring a biological ligand, there are brought into contact with each other in aqueous solution (a) a ligand to be measured, (b) an enzyme or a combination of an enzyme and a macromolecular substance, and (c) either a combination of an antibody against the above ligand and an antibody against the above enzyme, or a combination of an antibody against the above ligand, an antibody against the above enzyme and a macromolecular substance, determining the residual activity of the enzyme and utilising the result obtained as a measure of the ligand.

In our EP-A-0124366, there is disclosed a method of measuring a biological ligand, characterised by allowing there to coexist in an aqueous medium:

(A) a biologically active composition comprising firstly an immobilised antibody phase whose antibody is capable of reacting with a ligand (1) to be measured or an immobilised phase of a biological ligand (2) which is capable of reacting with said antibody and secondly an immobilised biotinyl enzyme phase or immobilised biotinyl enzyme inhibitor phase,

(B) a water-soluble material combination of whichever of said ligand (2) or said antibody on the one hand and a biotinyl enzyme inhibitor or a biotinyl enzyme on the other hand are absent from the biologically active composition, the biotinyl enzyme inhibitor then being one which is capable of reacting with the biotinyl enzyme of the biologically active composition or the biotinyl enzyme then being one which is capable of reacting with the biotinyl enzyme inhibitor of the biologically active composition, and

(C) the ligand (1) to be measured,

and measuring the residual biotinyl enzyme activity or the residual biotinyl enzyme inhibitory activity of said biologically active composition or said aqueous solution and utilising the result as a measure of the ligand.

In our EP-A-0132292, there is described a method of measuring a biological ligand which comprises allowing there to coexist in an aqueous medium a system comprising (A) a ligand (1) to be measured, (B) a combination of a biotinyl enzyme inhibitor and a ligand (2) having an antigenic determinant which is also an antigenic determinant of ligand (1) and (C) an antibody capable of reacting with the said antigenic determinant, contacting said system with a biotinyl enzyme, measuring the residual biotinyl enzyme activity and utilising the measurement thereby obtained as a measure of the biological ligand.

Finally, the present inventors have proposed in EP-A-0144176 (European patent Application No.84307834.6) a method of measuring a biological ligand which comprises allowing there to coexist in an aqueous medium a system comprising (A) a ligand (1) to be measured, (B) either a combination (1) of an antibody against the ligand (1) and an enzyme capable of acting on a water-insoluble macromolecular

2

EP 0 152 305 B1

substance or a combination (2) of a said enzyme and a ligand (2) having an antigenic determinant which is an antigenic determinant of said ligand (1), which combination (2) is present in the aqueous medium together with a said antibody, and (C) a said macromelecular substance, determining the residual activity of the enzyme after it has acted on said macromelecular substance and utilising the measurement thereby obtained as a measure of the biological ligand.

This last method has now been investigated further and it has been found that when an amylase is employed as the enzyme, the ligand (1) can be detected with very high sensitivity. However, when using a sample derived from a higher animal, since the sample usually contains an amylase, the reading for a blank is high, thereby leading to large errors. Hence investigations have been carried out in order to lower the value of blank determinations, i.e. to cancel out the effect of the amylase originally present in the sample. However, when the amylase in a sample was previously inactivated. for example by heating our by treating with an acid or an alkali, some ligands in the sample were also denatured or decomposed simultaneously. On the other hand, when the sample was diluted in order to lower the blank value, the sensitivity of the method for detecting the ligand (1) was also lowered. In addition, since such additional steps make the operation more complicated, their use is contrary to the general aim of providing a simple method for measuring the ligand (1).

It is an object of this invention to provide a method of measuring a biological ligand in a sample containing an amylase in which the effect of amylase in the sample is reduced without sacrificing the simplicity and high sensitivity of the method.

According to the present invention, there is provided a method of measuring a biological ligand in a sample containing an amylase from a higher animal, which comprises bringing into contact with each other in an aqueous medium a sample derived from a higher animal containing (A) a ligand (1) to be measured and (B) the amylase (1) from the higher animal, (C)(a) an antibody (1) against said ligand (1) to which is bound an amylase (2) not contained in said sample or (b) an antibody (1) against said ligand (1) and a biological ligand (2) to which is bound an amylase (2) not contained in said sample, said ligand (2) having at least one antigenic determinant in common with said ligand (1), and (D) an amylase inhibitor whose inhibitory activity against said amylase (1) is stronger than that against said amylase (2), the said antibody reacting (a) with said ligand (1) or (b) with said ligand (1) and with said ligand (2) to which said amylase (2) is covalently coupled and the said amylase inhibitor reacting with said amylase (1), digesting with said amylase (2) (E) a water insoluble macromolecular substrate of said amylase (2) and determining the residual activity of said amylase (2) and utilising the measurement thereby obtained a s a measure of said ligand (1),

and excluding the method when applied to measurement of theophylline in a sample of human serum containing amylase by bringing into contact with each other the serum sample, anti-theophylline mouse IgG as an antibody against the theophylline, a covalently coupled combination of α-amylase and theophylline and anti-human amylase goat IgG, as an inhibitor of amylase whose activity against amylase in the sample is stronger than that against α-amylase in the covalently coupled combination for said anti-theophylline mouse IgG to react with the theophylline of the sample and the theophylline to which the α-amylase is covalently coupled and for the anti-human amylase goat IgG to act on amylase form said sample to inhibit its activity,digesting starch with the α-amylase and determining the residual activity of the α-amylase in the covalently coupled combination and utilising the measurement obtained as a measure of the theophylline in the human serum sample.

The amylase (1) derived from a higher animal contained in a sample being measured by the method of the invention is, for example, pancreatic amylase or salivary amylase of any higher animal. The nature of the sample is not significant to the method of this invention and it may be, for example serum, plasma or urine. In the case of serum and urine, no pretreatment is necessary.

The aim of the method of the invention is to measure a ligand (1). The ligand (1) is a substance having one or more antigenic determinants, and may be, for example, a hormone derived from one of various endocrine glands, a plasma protein such as immunoglobulin, albumin and ferritin, a viral antigen such as HB antigen, bacteria, α-fetoprotein or a carcinoembryonic antigen.

The ligand (2), when used, also has one or more antigenic determinant(s) of ligand (1). All antigenic determinants of ligand (2) may be antigenic determinants of ligand (1), and accordingly, ligand (2) may be identical with ligand (1).

The antibody (1) used is an antibody against the ligand (1). When a ligand (2) is employed, this antibody should also be an antibody against ligand (2). Thus, in such a case, the antibody should be an antibody against the aforementioned common antigenic determinant. The antibody may be, for example, a fragment of immunoglobulin, for example F(ab')₂, Fab' or Fab, or such a fragment modified by introduction of a DNP group, acetyl group, biotinyl group or nitro group.

3

Such an antibody may be isolated by a conventional method of producing an antibody. For example, ligand (1), ligand (2) (if to be used) or a covalently coupled conjugate of either of these ligands and a protein material is injected once or several times into the subcutaneous region of the back, foot pad or femoral muscle of a warm-blooded animal such as a rabbit, goat, horse, guinea pig or chicken, in an amount of from 0.3 to 2 mg per kg body weight together with an adjuvant, and the required antibody is produced in a humoral fluid such as serum. This humoral fluid may be used as such as the antibody source. However, the antibody is preferably separated by a conventional isolation method for an immunoglobulin.

Alternatively, the antibody may be produced as a monoclonal antibody. In this case, one of the above antigens is injected several times into the abdominal cavity of a mouse together with an adjuvant, and its spleen is excised. A spleen cell is fused with a mouse myeloma cell by a conventional method involving the use of polyethylene glycol. The hybridoma thus obtained is cultured and cloned, and a cell capable of producing the desired antibody is obtained. This cell is injected into the abdominal cavity of a mouse, and multiplied. Then, ascites are collected, and the desired antibody is separated from the ascites.

If the antibody (C) is not covalently coupled to the amylase (2),

and if the amylase (2) activity does not appreciably vary as a result of the reaction of the antibody with the ligand (2) portion of the covalently coupled combination of ligand (2) and amylase (2),

a macromolecular compound is preferably covalently coupled to the antibody prior to use. Preferred macromolecular compounds are those which are water-soluble and have molecular weights preferably greater than 100,000 daltons. Examples of such macromolecular compounds include polysaccharides and derivatives thereof, for example soluble dextran, carboxymethyl dextran, dextran with induced amino group and amylose, proteins for example gelatin, haemocyanin and ferritin, and polyethylene glycol. The macromolecular compound may be covalently coupled to the antibody by the coupling method to be described later.

The amylase (2) does not exist initially in the sample under test and it is employed covalently coupled to the above antibody (C) or to the ligand (2). This amylase (2) is, for example α-amylase, β-amylase or glucoamylase. The choice of amylase (2) will vary according, for example, to the nature of the sample under test and the nature of the amylase inhibitor to be used, as will be described hereinafter. More specifically, it is for example diastase or β-amylase derived from malt, taka-diastase derived from a mould, or amylase derived from a bacterium of genus Bacillus.

Amylase (2) is covalently coupled to the antibody (1) or to a ligand (2). When it is to be coupled with the antibody, the ligand (2) is not used. On the other hand, when it is to be coupled with the ligand (2), the antibody is used in its natural form or covalently coupled with a macromolecular compound as mentioned hereinbefore.

The method of coupling the amylase (2) with the antibody (1) or the ligand (2) will generally depend on the functional groups of both substances. Such functional groups include, amino groups, carboxyl groups, hydroxyl groups, thiol groups, imidazole groups and phenyl groups. The binding of amino groups, may be carried out by many methods including the diisocyanate method, the glutaraldehyde method, the difluorobenzene method, and the benzoquinone method. For binding an amino group and a carboxyl group, it is possible to use the peptide-binding method for binding a carboxyl group to a succinimido ester group, the carbodiimide method and the Woodward reagent method. The periodate oxidation method (Nakane method) in which a bridge is formed between amino groups and sugar chain forms may also be utilised for this purpose. When using a thiol group, a carboxyl group, for example, is first converted to a succinimido ester group, and this ester group is then allowed to react with cysteine to introduce a thiol group, and both thiol groups which are present are bound by using a thiol-reactive bifunctional cross-linking reagent, for example phenylene-bismaleimide. Methods utilising a phenyl group include the diazotization method and the alkylation method. In addition to the foregoing, there may be considered one of various methods described in "Methods in Immunology and Immunochemistry" (C.A. Williams et al., 1976, Academic Press N.Y.) and "Koso Meneki Sokutei-ho" (E. Ishikawa et al., 1978, Igaku-shoin (Japan)). The molar ratio of the combination is not limited to 1:1, and other suitable ratios can be selected. After the binding reaction, the combination produced is purified by gel filtration, ion-exchange chromatography and affinity chromatography, and lyophilized, if necessary.

When the amylase (2) has been coupled with the antibody (1), the antibody is allowed to contact the ligand (1) to be measured. When the amylase (2) has been coupled with the ligand (2), the antibody is allowed to contact the ligand (1) and the ligand (2) portion of the combination of ligand (2) and amylase (2). In the latter case, the order of the contacting is immaterial, and either of the ligand (1) and the combination of ligand (2) and amylase (2) may first be allowed to make contact with the antibody. Of course, both the ligand (1) and the combination of ligand (2) and amylase (2) may be allowed to make contact with the antibody at the same time. The temperature of the aqueous solution being used is usually kept at from 20 to 45°C, and the pH is usually kept at from 4 to 9. In order to keep the pH constant, a buffer solution such as a phosphate buffer solution or an acetate buffer solution may be employed. Since the quantities of the covalently coupled combination of ligand (2) and amylase (2) and the antibody will differ, for example according to their identities, the character of the ligand (1) and the contacting conditions, the quantities are preferably determined in a preliminary test.

If the antibody (1) is not employed covalently coupled with the amylase (2) and if the activity of the amylase (2) does not appreciably vary as a result of the reaction of the antibody with the ligand (2) portion of the covalently coupled combination, a second antibody (3) may be introduced to react with the antibody (1) which is covalently coupled to the ligand (2) portion of the combination thereof with amylase (2). The second antibody (3) may be prepared by following one of the procedures previously described herein.

On the other hand, when the antibody (1) is used in covalently coupled combination the amylase (2), the sensitivity is raised by one or two orders by adding another antibody (2) which is an antibody against a different antigenic determinant of the ligand (1) from that which to which the first antibody is an antibody. This antibody may also be produced by following a conventional method as aforedescribed. Since the antibody in the humoral fluid is a mixture of antibodies recognising various antigenic determinants, it is necessary for them to be separated. The separation may be carried out by affinity chromatography. For example, the ligand (1) is digested by an enzyme or a chemical reagent, and the fragments produced are separated by gel filtration or ion-exchange chromatography. Each fragment is immobilised on a carrier, and hence a column for affinity chromatography is prepared. The above mixture of antibodies is separated by using this column. It is sufficient for the mixture of antibodies to be separated into two groups The method of producing a monoclonal antibody by using the cell fusion technique described previously is suitable for the production of such a specific antibody. A water-soluble macromolecular compound may be covalently coupled to the antibody in order to raise the sensitivity. A molecular weight of greater than 1,000 is preferable. Examples of such macromolecular compounds include proteins such as albumin and haemocyanin, polysaccharides, polyethylene glycol and polynucleotides. The coupling method used may be one of those previously described for binding of amylase (2). A second antibody (4) may be coupled to this antibody (2) in order to raise the sensitivity. The second antibody (2) may also be prepared by following a method as previously described. This second antibody (4) may be added to the antibody (2) prior to contacting with the ligand (1) or at the same time.

In the method of this invention, the amylase (1) derived from a higher animal and present in the sample under test is brought into contact with an amylase inhibitor whose inhibitory activity against amylase (1) is stronger than that against amylase (2).

The most desirable amylase inhibitor inactivates all amylases (1) present in the sample and does not inhibit amylase (2). However, it is sufficient for practical purposes that the amylase inhibitor inactivates the main amylase (1) in the sample. The inactivation is maintained throughout the determination, and the activity of amylase (1) may be restored after the determination.

Examples of such amylase inhibitors include the amylase inhibitor derived from wheat which inhibits both salivary amylase and pancreatic amylase (M.D. O'Donnell et al., Biochim, Biophys. Acta, vol. 422, pp159-169 (1976)), amylase inhibitor Sain derived from wheat which preferentially inhibits salivary amylase (Japanese Patent Application Kokai 85899/1983) and amylase inhibitor AI-B produced by an actinomycetes of genus Streptomyces which preferentially inhibits pancreatic amylase (Japanese Patent Application Kokai 2684/1982). Moreover, when amylase (1) contained in the sample is injected into a foreign animal to produce the antibody, this antibody may also be utilised as the amylase inhibitor. These amylase inhibitors may be used jointly.

The temperature and the pH of the aqueous solution while the amylase inhibitor is in contact with the amylase (1) are usually chosen to be the same as the temperature and pH conditions under which contacting of the ligand (1) with the antibody is carried out. The amount of amylase inhibitor used depends on its character, the character and the amount of amylase (1), the character of amylase (2) and the contacting conditions. The amount used is preferably determined by a preliminary test. The amylase inhibitor is usually added prior to the addition of the water-insoluble macromolecular substance. However, since the inhibitory action of the amylase inhibitor is generally much faster than the decomposition rate of the substrate by the amylase (1), the amylase inhibitor may be added together with the macromolecular substrate (E)

The final constituent of the system to be discussed is water-insoluble macromolecular substrate (E) capable of digestion by amylase (2). The bound amylase (2) will usually already be present in the reaction solution, although it may be separated from it when the water-insoluble macromolecular substrate is added.

The macromolecular substance (E) is able to be digested by the amylase (2), and it is usually a substrate for amylase (2).

The macromolecular substrate (E) is characterised by its property of insolubility in water. This means that most of the contacting of the macromolecular substrate with the bound amylase (2) is carried out at the boundary between solid and solution, and as a result, steric hindrance of macromolecule(s) bound to the amylase (2) largely occurs. This is supported by an experiment which has been carried out where a pentose and an insoluble starch were digested by an $\alpha$-amylase which was in its natural form or which had previously been allowed to combine with a macromolecule. As a result of the experiment, enzyme activity was hardly lowered by the combination of the macromolecule in the case of the pentose, while it was considerably lowered in the case of the insoluble starch. The macromolecular substances include insoluble starches. When a macromolecular substrate which is available is water-soluble, it may be subjected to insolubilisation. The insolubilisation may be carried out by covalent coupling with an insoluble carrier material or by subjecting the water soluble macromolecule substance to polymerisation. For example, the water-soluble macromolecular substance is entrapped with agarose gel.

The conditions for the enzyme reaction will be determined by the amylase (2) employed.

After the reaction, the residual amylase (2) activity is determined by detecting the changes in the reaction mixture, such as the increase of a decomposition product, the decrease of the raw material, etc. Any of the methods described in the earlier patent applications discussed hereinabove may be used as appropriate. Some of the methods will be apparent from the examples which follow.

By carrying out the method of this invention, a biological ligand (1) in a sample derived from a higher animal and containing an amylase such as human serum can be detected and determined in high sensitivity and in high specificity. The operation of this method is simple, and a biological ligand (1) can easily and inexpensively be determined. There is no limitation to the type of ligand (1) to be measured and the present method is particularly suitable for determining a ligand having a relatively high molecular weight. When using a covalently coupled combination of the antibody and the amylase (2), since the ligand (1) is used only as an antigen which is necessary to produce the antibody, the amount of the ligand (1) may be very small. Accordingly, this method is effective when only a small amount of ligand (1) can be obtained or when the ligand (1) is extremely expensive.

Hitherto a method of measuring a lower hapten such as digoxin or theophylline bound to an enzyme has been reported. However, no method of measuring an extremely small amount of an antigen of the order of ng/ml level, the antigen having a molecular weight of more than 10,000, has bee reported. The present invention offers a highly sensitive method of determining a macromelecular antigen, and it has extended the level of the hitherto employed determinations from g/ml to ng/ml.

Moreover our EP-A-0144176 referred to earlier herein and as published on 12th June 1985, discloses a method of measuring theophylline (1) in a sample of human serum containing amylase (2) which comprises bringing into contact with each other the serum sample containing the theophylline, anti-theophylline mouse IgG as antibody (3) against the theophylline, a covalently coupled combination of $\alpha$-amylase and theophylline (4) and anti-human amylase goat IgG as an amylase inhibitor (5) whose inhibiting activity against amylase (2) is stronger that that against said $\alpha$-amylase for said antibody (3) to react with the theophylline of said sample and the theophylline to which the $\alpha$-amylaseis covalently coupled and for said amylase inhibitor (5) to react with said amylase (2), digesting starch with the $\alpha$-amylase and determining the residual activity of said $\alpha$-amylase and utilising the measurement thereby obtained as a measure of the theophylline in said human serum sample.

The following Examples illustrate this invention:

EXAMPLE 1

(i) Preparation of CHM-induced $\alpha$-Amylase

1 mg of Bacillus subtilis $\alpha$-amylase was dissolved in 1 ml of 0.1 M phosphate buffer solution of pH 6.3 containing 10 mM o-phenanthroline. 100 $\mu$l of a solution of 4-maleimidomethyl cyclohexane-1-carboxylic acid N-hydroxy succinimide ester (CHMS) in dimethylformamide (DMF) in a concentration of 2 mg /ml was added and the mixed solution obtained was allowed to stand at ambient temperature for 1 hour. The reaction mixture was introduced into Sephadex (Registered Trade Mark) G-25 column, and gel filtration was carried out using 0.1 M phosphate buffer solution of pH 6.3. The void fractions were collected to obtain the desired CHM-induced amylase.

ii) Preparation of SH-induced $\alpha$-Fetoprotein

5 mg of α-fetoprotein were dissolved in 0.1 M phosphate buffer solution of pH 6.0 containing 5 mM EDTA (ethylenediaminetetraacetate). 100 μl of a solution of S-acetylmercaptosuccinic anhydride (SAMS) in DMF in a concentration of 9 mg/ml were added to this, and allowed to react at 37°C for 1 hour. 110 μl of 1 M hydroxylamine aqueous solution of pH 7.5 were added to the reaction mixture, and the mixture obtained was warmed at 37°C for 30 minutes. Subsequently, gel filtration of the reaction mixture using Sephadex G-25 was carried out and the void fractions were collected to obtain the object SH-induced α-fetoprotein.

iii) Preparation of Amylase - α-Fetoprotein covalently coupled combination

The above CHM-induced amylase solution was mixed with the SH-induced α-fetoprotein solution. The mixture obtained was concentrated to 1 ml, and allowed to react at 4°C overnight. The reaction, solution obtained was introduced into a Sephacryl S-300 column (Sephacryl is a Registered Trade Mark), and gel filtration was carried out by using 20 mM phosphate buffered saline solution of pH 7.0. The fractions containing the covalently coupled combination of amylase and α-fetoprotein in a ratio of 1:3 were collected and combined.

iv) Measurement of α-Fetoprotein

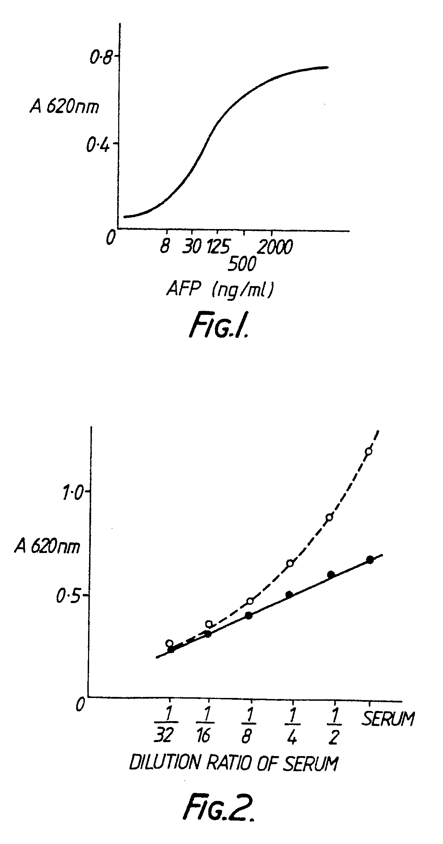

50 μl samples of human α-fetoprotein solution whose α-fetoprotein concentration was in the range of 0-2000 ng were each mixed with 50 μl of the combined solution prepared in step iii) to which 5% by weight of polyethylene glycol 6,000 had been added. 50 μl of a solution containing 100 μg/ml of a mixture of α-amylase inhibitors derived from wheat and 8 μg/ml anti-human-α-fetoproteingoat IgG were also added to each mixture, and allowed to react for 20 minutes. 1.0 ml of a suspension of blue starch (made by Pharmacia, Diagnostics, A.B.) was added to each reaction mixture, and allowed to react at 37° for 20 minutes. The enzyme reaction was terminated by adding 1 ml of 0.5 N NaOH. Each mixture was stirred, and then centrifuged at 3,500 rpm for 2 minutes. The absorbance of 620 nm of the supernatant was measured in each case. The relationship between the α-fetoprotein concentration (designated as AFP on the abscissae) and the absorbance (designated at A on the ordinate axis) thus obtained is shown in Figure 1 of the accompanying drawings.

Subsequently, a human serum was diluted with a phosphate buffered saline solution (PBS) to prepare a $2^n$ dilution series, and 50 μl samples of each series were placed in small test tubes. 50 μl of the combination solution prepared in step iii) to which 10% by weight of polyethylene glycol 6,000 had been added and 50 μl of 8 g/ml anti-human- α-fetoprotein goat IgG solution containing or not containing 100 μg/ml of the amylase inhibitor mixture derived from wheat were added to each test tube, and reaction was allowed to occur at 37°C for 20 minutes. 1.0 ml of blue starch suspension was added to each reaction mixture, and allowed to react at 37°C for 20 minutes. The enzyme reaction was terminated by adding 1 ml of 0.5 M (N) NaOH. The mixture obtained was stirred, and then centrifuged at 3,500 rpm for 2 minutes. The absorbance of the supernatant at 620 nm was measured. The relation between the dilution ratio of the serum and the absorbance thus obtained is shown in Figure 2 of the accompanying drawings in which absorbance is plotted against dilution ratio. In the figure, solid circles indicate those cases in which the amylase inhibitor was added, and open circles indicate those cases in which it was not added. As can be seen from the figure, when the inhibitor was not added, the absorbance was increased by the amylase in the serum. Even at a dilution ratio of 32 times, the abosrbance was slightly higher. On the other hand, when the inhibitor was added, the α-fetoprotein concentration could be determined without either concern for dilution or need to use a blank.

EXAMPLE 2

i) Preparation of CHM-induced α-Amylase

5 mg of Bacillus substilis α-amylase were dissolved in 1 ml of 0.1 M phosphate buffer solution of pH 6.3. 100 μl of CHMS DMF solution (CHMS concentration of 2mg/ml) were added to this, and the mixture obtained was allowed to stand at ambient temperature for 1 hour. The reaction mixture was introduced into a Sephadex G-25 column, and gel filtration was carried out using 0.1 M phosphate buffer solution of pH 6.3. The void fractions were collected to provide the desired CHM-induced amylase.

ii) Preparation of Human IgG F(ab')₂

10 mg of human IgG were dissolved in 2 ml of 0.1 M acetate buffer solution of pH 4.0 containing 1 mM EDTA. 300 $\mu$g of pepsin were added to this, and stirring at 37°Cwas effected for 18 hours. The solution obtained was adjusted to pH 6.0 by adding 0.1 N NaOH, and introduced into a Sephacryl S-300 column which had previously been equilibrated with 0.1 M phosphate buffered 1 mM EDTA solution of pH 6.3, and eluted by means of the above phosphate buffer solution. Peak fractions corresponding to molecular weights of about 100,000 were collected and combined, and concentrated to 1 ml to obtain the desired human IgG F(ab')$_2$.

iii) Preparation of Covalently Coupled Combination of Amylase and Human IgG Fab'

1 ml of 0.1 M phosphate buffered 1 mM EDTA solution of pH 6.0 containing 6 mg of the above human IgG F(ab')$_2$ was mixed with 100 $\mu$l of aqueous 2-mercaptoethylamine hydrochloride solution having a concentration of 10 mg/ml and stirring at 37°C for 90 minutes was effected. Gel filtration was carried out using a Sephadex G-25 column which had previously been equilibrated with 0.1 M phosphate buffer solution of pH 6.3 and unreacted 2-mercaptoethylamine was removed to obtain SH-Fab'. 2 mg of CHM-induced $\alpha$-amylase prepared in step i) were added to the SH-Fab' solution thus obtained, and reaction at 37°C was allowed to occur for 90 minutes. Subsequently, the reaction mixture was subjected to gel filtration using a Sephacryl S-300 column which had been equilibrated with 0.1 M acetate buffered 5 mM calcium chloride solution of pH 6.0, and fractions corresponding to a molecular weight of greater than 200,000 were collected and combined. The combined fractions were concentrated to obtain the desired covalently coupled combination.

iv) Measurement of Human IgG

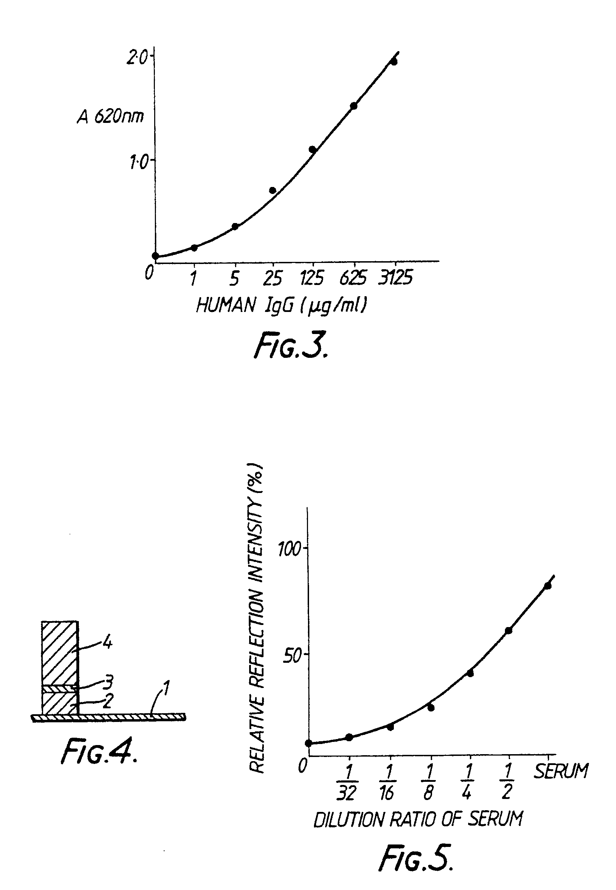

50 $\mu$l samples of a human IgG solution whose concentration of IgG was in the range of 0-3125 $\mu$g/ml were each mixed with 50 $\mu$l of the combined solution prepared in the above step iii) to which 10% by weight of polyethylene glycol 6,000 had been added. 50 $\mu$l of a solution containing 100 $\mu$g/ml of the amylase inhibitor produced by Streptomyces viridosporus No.297-A2 FERM-P 5405 and 100 $\mu$g/ml of anti-human IgG goat IgG were added to this and reaction was allowed to occur at 37°C for 20 minutes. 1.0 ml of blue starch suspension was added to the reaction mixture, and further reaction was allowed to occur at 37°C for 10 minutes. The enzyme reaction was terminated by adding 1 ml of 0.5 N NaOH. The mixtures were stirred, and then centrifuged at 3,500 rpm for 2 minutes. The absorbance of the supernatant in each case was measured. The relationship between the human IgG concentration and the absorbance thus obtained is shown in Figure 3 of the accompanying drawings in which absorbance at 620 nm is plotted against human IgG concentration ( $\mu$g/ml).

EXAMPLE 3

i) Preparation of CHM-induced $\alpha$-Amylase

1 mg of Bacillus subtilis $\alpha$-amylase was dissolved in 1 ml of 0.1 M phosphate buffer solution of pH 6.3. 100 $\mu$l of 2 mg/ml CHMS DMF solution were added to this, and the mixture produced was allowed to stand at ambient temperature of 1 hour. The reaction mixture was introduced into a Sephadex G-25 column, and gel filtration was carried out by using 0.1 M phosphate buffer solution of pH 6.3. The void fractions were collected and combined to obtain the desired CHM-induced $\alpha$-amylase.

ii) Preparation of SH-induced IgE

5 mg of Human IgE were dissolved in 0.1 M phosphate buffer solution of pH 7.5 containing 5 mM EDTA. 100 $\mu$l of 9 mg/ml SAMS DMF solution were added to this, and reaction at 37°C was allowed to occur for 1 hour. 110 $\mu$l of 1 M hydroxylamine aqueous solution of pH 7.5 were added to the reaction mixture, which was warmed at 37°C for 30 minutes. Subsequently, gel filtration of the reaction mixture using Sephadex G-25 was carried out, and the void fractions obtained were collected and combined to obtain the object SH-induced IgE.

iii) Preparation of Covalently Coupled Combination of Amylase and IgE

The above CHM-induced $\alpha$-amylase solution was mixed with the SH-induced human IgE solution. The mixture obtained was concentrated to 1 ml, and allowed to react at 4°C overnight. The reaction mixture was then introduced into a Sephacryl S-300 column, and gel filtration was carried out using 20 mM phosphate buffered saline solution of pH 7.0. The fractions containing the covalently coupled combination whose molar ratio was 1:2 were collected and combined.

iv) Measurement of Human IgE

50 $\mu$l samples of this combined solution (concentration of 10 $\mu$g/ml) were added to 50 $\mu$l of $2^n$ diluted human serum samples with PBS, and in order to inhibit human serum amylase, 50 $\mu$l of 500 $\mu$g/ml anti-human amylase goat IgG were added to each mixture. 50 $\mu$l of 10 $\mu$l/ml anti-human IgE goat IgG were further added to each mixture, and reaction was allowed to occur at 37°C for 30 minutes. 100 $\mu$l of each reaction solution were dropped onto a laminate film as shown in Figure 4 of the accompaying drawings which consisted of polystyrene film 1, an anion exchange resin layer 2, a reflection layer 3 and a blue starch layer 4. The amylase activity at ambient temperature after 20 minutes was measured by using a reflectometer. The relation between the human IgE concentration and the reflection intensity thus obtained is shown in Figure 5 of the accompanying drawings in which relative reflection intensity is plotted as a percentage against the dilution ratio of the test serum.

EXAMPLE 4

i) Preparation of CHM-induced $\alpha$-Amylase

5 mg of Bacillus subtilis $\alpha$-amylase were dissolved in 1 ml of 0.1 M glycerophosphate buffer solution of pH 6.3 containing 10 mM o-phenanthroline. 100 $\mu$l of 2 mg/ml CHMS DMF solution were added to this, and the mixture obtained was allowed to stand at ambient temperature for 1 hour. The reaction mixture was introduced into a Sephadex G-25 column, and gel filtration was carried out by using 0.1 M phosphate buffer solution of pH 6.3. The void fractions were collected and combined to yield the desired CHM-induced $\alpha$-amylase.

ii) Preparation of Anti-Human $\alpha$-Fetoprotein Goat IgG F(ab')$_2$

10 mg of anti-human $\alpha$-fetoprotein goat IgG were dissolved in 2 ml of 0.1 M acetate buffer solution of pH 4.0. 300 $\mu$g of pepsin were added to this, and the solution produced was stirred at 37°C for 18 hours. The solution was adjusted to pH 6.0 by adding 0.1 N NaOH, and introduced into a Sephacryl S-300 column which had previously been equilibrated with 0.1 M phosphate buffered 1 mM EDTA solution of pH 6.3, and eluted with the above phosphate buffer solution. Peak fractions corresponding to the molecular weight of about 100,000 were collected and combined, and concentrated to 1 ml to obtain the desired anti-human $\alpha$-fetoprotein goat IgG F(ab')$_2$.

iii) Preparation of Covalently Coupled Combination of Amylase and Anti-Human $\alpha$-Fetoprotein Goat IgG Fab'

1 ml of 0.1 M phosphate buffered 1 mM EDTA solution of pH 6.0 containing 6 mg of the above anti-human $\alpha$-fetoprotein goat IgG F(ab')$_2$ was mixed with 100 $\mu$l of an aqueous solution of 2-mercapto-toethylamine hydrochloride (concentration, 10 mg/ml) and stirred at 37°C for 90 minutes. Gel filtration was carried out using a Sephadex G-25 column which had previously been equilibrated with 0.1 M phosphate buffer solution of pH 6.3 and unreacted 2-mercaptoethylamine was removed to obtain SH-Fab'. 2 mg of CHM-induced $\alpha$-amylase prepared in step i) were added to the SH-Fab' solution thus obtained, and allowed to react at 37°C for 90 minutes. Subsequently, this reaction mixture was separated by gel filtration using a Sephacryl S-300 column which had been equilibrated with 0.1 M acetate buffered 5 mM calcium chloride solution of pH 6.0, and the fractions corresponding to molecular weights of greater than 200,000 were collected. These fractions were concentrated and combined to obtain the desired covalently coupled combination.

iv) Measurement of $\alpha$-Fetoprotein

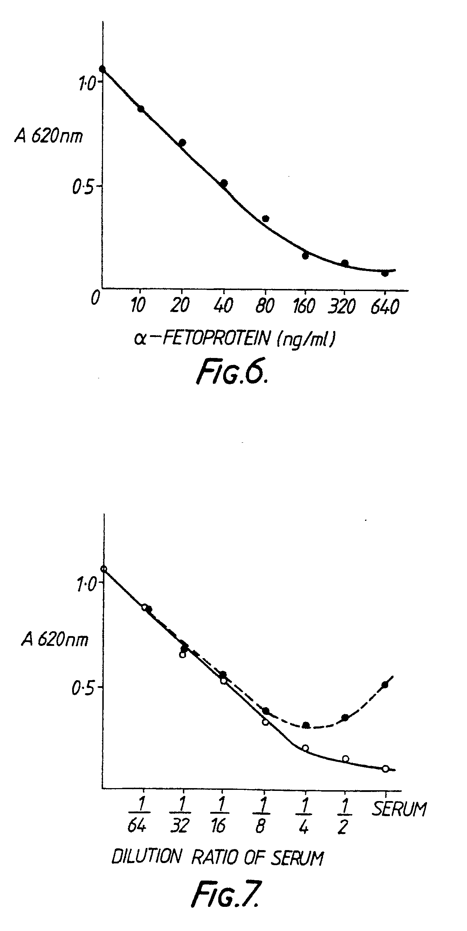

100 $\mu$l samples of a human $\alpha$-fetoprotein solution whose concentration was in the range of 0-2000 ng were each mixed with 50 $\mu$l of the combined solution prepared in the above step iii) to which 100 $\mu$l/ml of the amylase inhibitor produced by Streptomyces viridisporus No.297-A2 FERM-P 5405 and 7% by weight of polyethylene glycol 6000 had been added, and reaction was allowed to occur at 37°C for 30 minutes. 1.0 ml of blue starch suspension was added to each reaction mixture, and further reaction was allowed to occur at 37°C for 30 minutes. The enzyme reaction was termined by adding 1 ml of 0.5 N NaOH. The reaction mixtures were stirred, and then centrifuged at 3,5000 rpm for 2 minutes. The absorbance at 620 nm of the supernatant was measured in each case. Figure 6 of the accompanying drawings shows the relationship between the human $\alpha$-fetoprotein concentration and the absorbance thus obtained.

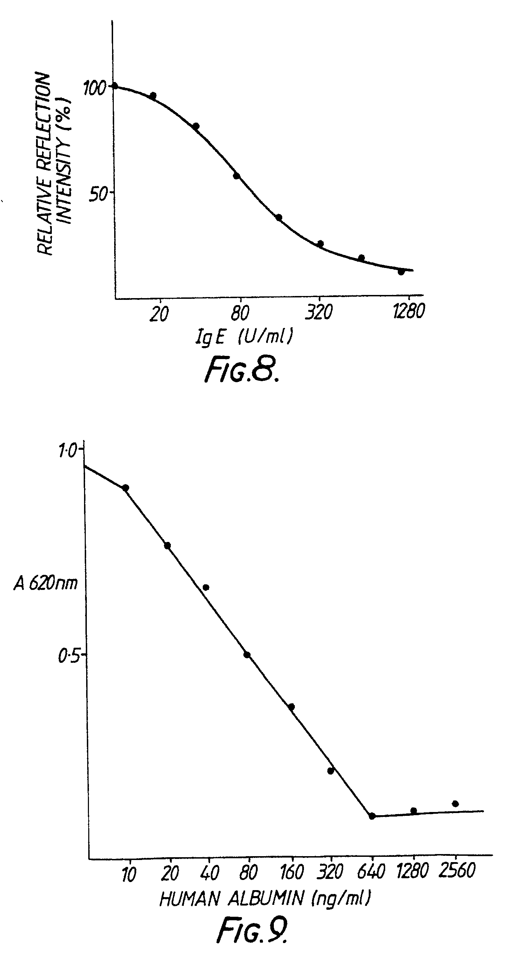

Subsequently, 100 $\mu$l of a human serum containing human $\alpha$-fetoprotein were diluted with 20 mM glycerophosphate buffer solution of pH 6.0 to prepare $2^n$ dilution series. 100 $\mu$l samples of each member of the series were placed in a small test tube, and mixed with 100 $\mu$l of the combination solution prepared in step iii) to which the amylase inhibitor produced by Streptomyces viridosporus No.297-A2 FERM-P 5405 and 7% by weight of polyethylene glycol 6,000 had been added. The mixtures were warmed at 37°C for 30 minutes. 1.0 ml of blue starch suspension was added to each reaction mixture, and further reaction took place at 37° for 30 minutes. The enzyme reactions were terminated by adding 1 ml of 0.5 N NaOH. The mixtures obtained were stirred, and then centrifuged at 3,500 rpm for 2 minutes. The results are shown in Figure 7 of the accompanying drawings in which absorbance at 620 nm of the supernatant is plotted against dilution ratio. In Figure 7, open circles indicate the cases in which the amylase inhibitor was added, and solid circles indicate the cases in which the amylase inhibitor was not added. As can be seen from Figure 7, when the amylase inhibitor was not added, the absorbance was increased by the amylase in the serum. In contrast, when the amylase inhibitor was added, the $\alpha$-fetoprotein could be determined without the dilution being a matter of concern or a blank needing to be used.

## EXAMPLE 5

### i) Preparation of SPDP-induced $\alpha$-amylase

1 mg of Bacillus subtilis $\alpha$-amylase was dissolved in 0.1 M glycerophosphate buffer solution of pH 7.5 containing 10 mM o-phenanthroline. 100 $\mu$l of 1 mg/ml N-succinimidyl-3-(2-pyridyldithio) propionic acid (SPDP) DMF solution were added to this, and the mixture obtained was allowed to stand at ambient temperature for 30 minutes. The reaction mixture was introduced into a Sephadex G-25 column which had previously been equilibrated with 0.1 M glycerophosphate buffer solution of pH 7.5, and gel filtration was carried out to obtain 1 mg of SPDP induced amylase.

### ii) Preparation of Anti-Human IgE Goat IgG F(ab')$_2$

10 mg of anti-human IgE goat IgG were dissolved in 2 ml of 0.1 M acetate buffer solution of pH 4.0. 300 $\mu$g of pepsin were added to this, and stirring was carried out at 37°C for 18 hours. The solution obtained was adjusted to pH 6.0 by adding 0.1 N NaOH, and introduced into a Sephacryl S-300 column which had previously been equilibrated with 0.1 M phosphate buffered 1 mM EDTA solution of pH 6.3, and eluted using the above phosphate buffer solution. Peak fractions corresponding to a molecular weight of about 100,000 were collected and combined, and concentrated to 1 ml to provide the desired anti-human IgE goat IgG F(ab')$_2$.

### iii) Preparation of Covalently Coupled Combination of Amylase and Anti-Human IgE Goat IgG Fab'

1 ml of 0.1 M phosphate buffered 1 mM EDTA solution of pH 6.0 containing 6 mg of the above anti-human IgE goat IgG F(ab')$_2$ was mixed with 100 $\mu$l of 10 mg/ml 2-mercaptoethylamine hydrochloride aqueous solution. The mixture obtained was stirred at 37°C for 90 minutes. Gel filtration using a Sephadex G-25 column which had previously been equilibrated with 0.1 M glycerophosphate buffer solution of pH 7.5 was carried out, and SH-Fab' was obtained by removing unreacted 2-mercaptoethylamine. 2 mg of SPDP-induced $\alpha$-amylase prepared in step i) were added to the SH-Fab' solution thus obtained and allowed to react at 4°C for 18 hours. Subsequently, this reaction mixture was separated by gel filtration using a Sephacryl S-300 column which had been equilibrated with 0.1 M acetate buffered 5 mM calcium chloride solution of pH 6.5, and the fractions corresponding to molecular weights of greater than 200,000 were collected and combined. the fractions were concentrated to obtain the desired covalently coupled combination.

### iv) Measurement of Human IgE

50 µl samples of a human IgE solution whose concentrations were in the range of 0-1280 U/ml were each mixed with 50 µl of the combined solution prepared in the above step iii) to which the amylase inhibitors mixture derived from wheat and 7% by weight of polyethylene glycol 6000 had been added, the former in a concentration of 100 µg/ml and reaction was allowed to occur for 30 minutes. 50 µl of this reaction solution were dropped on a laminate film as shown in Figure 4. The amylase activity at ambient temperature was measured after 20 minutes by using a reflectometer. Figure 8 of the accompanying drawings shows the relationship between the human IgE concentration and the reflection intensity thus obtained.

Subsequently, IgE concentrations of 5 sera samples were measured by the method of the invention and by the conventional sandwich EIA method. The results obtained are shown in the following Table.

## T a b l e

### IgE Concentration

| Serum | The Method of the invention | EIA method |
|---|---|---|
| 1 | 128 U/ml | 125 U/ml |
| 2 | 439 " | 451 " |
| 3 | 71 " | 68 " |
| 4 | 1190 " | 1120 " |
| 5 | 50 " | 51 " |

## EXAMPLE 6

i) Preparation of SPDP-induced $\alpha$-Amylase

1 mg of SPDP-induced $\alpha$-amylase was prepared from 1 mg of Bacillus subtilis amylase in the same manner as described in step i) of Example 5.

ii) Preparation of Anti-Human Albumin Goat IgG F(ab')$_2$

10 mg of anti-human ablbumin goat IgG were dissolved in 2 ml of 0.1 M acetate buffer solution of pH 4.0. 300 µg of pepsin were added to this, and the solution obtained was stirred at 37°C for 18 hours. The solution was adjusted to pH 6.0 by adding 0.1 N NaOH, and introduced into a Sephacryl S-300 column which had previously been equilibrated with 0.1 M phosphate buffered 1 mM EDTA solution of pH 6.3, and eluted using the above phosphate buffer solution. Peak fractions corresponding to a molecular weight of about 100,000 were collected and combined and then concentrated to 1 ml to obtain the desired anti-human albumin goat IgG F(ab')$_2$.

iii) Preparation of Covalently Coupled Combination of Amylase and Anti-Human Albumin Goat IgG Fab'

1 ml of 0.1 M phosphate buffered 1 mM EDTA solution of pH 6.0 containing 6 mg of the above anti-human albumin got IgG F(ab')$_2$ was mixed with 100 µl of 10 mg/ml 2-mercaptoethylamine hydrochloride aqueous solution, and stirring was effected at 37°C for 90 minutes. Gel filtration using a Sephadex G-25 column which had previously been equilibrated with 0.1 M glycerophosphate buffered solution of pH 7.5 was carried out, and unreacted 2-mercaptoethylamine was removed. In this way SH-Fab' was obtained. 1 mg of SPDP-induced $\alpha$-amylase prepared in step i) was added to the SH-Fab' solution thus obtained, and allowed to react at 4°C for 18 hours. Subsequently, this reaction mixture was separated by gel filtration using a Sephacryl S-300 column which had been equilibrated with 0.1 M acetate buffered 5 mM calcium chloride solution of pH 6.5, and fractions corresponding to molecular weights of greater than 200,000 were collected and combined. The fractions were concentrated to obtain the desired covalently coupled combination.

## iv) Measurement of Human Albumin

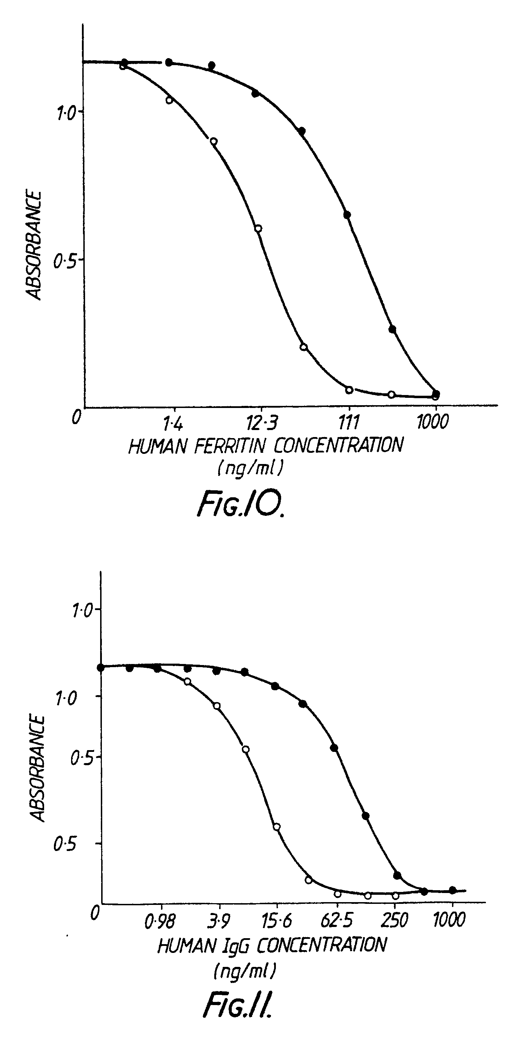

100 $\mu$l samples of human albumin solution whose concentrations were in the range of 0-2560 ng were placed in small test tubes, and each sample was mixed with 100 $\mu$l of the combined solution prepared in the above step iii) to which were added the amylase inhibitor produced by Streptomyces viridosporus No.297-A2 FERM-P 5404 (in a concentration of 100 $\mu$g/ml) and 10% by weight of polyethylene glycol 6,000. Then, the mixtures obtained were allowed to undergo reaction at 37°C for 30 minutes. 1.0 ml of blue starch suspension was added to each reaction mixture, and further reaction was allowed to occur at 37°C for 30 minutes. The enzyme reaction was terminated by adding 1 ml of 0.5 N NaOH. The mixtures were stirred, and then centrifuged at 3,500 rpm for 2 minutes. The results are shown in Figure 9 of the accompanying drawings in which absorbance at 620 nm of the supernatant was plotted on the ordinate axis against human albumin on the abcissae.

## EXAMPLE 7

### i) Preparation of CHM-induced $\alpha$-Amylase

CHM-induced $\alpha$-amylase was prepared from Bacillus subtilis $\alpha$-amylase in the same manner as described in step i) of Example 4.

### ii) Preparation of Anti-Human Ferritin Mouse Monoclonal IgG F(ab')$_2$

10 mg of anti-human ferritin mouse monoclonal IgG were dissolved in 2 ml of 0.1 M acetate buffered 3 mM EDTA - 0.1 M cysteine solution of pH 5.5. 300 $\mu$g of papain were added to this, and stirring at 37°C was effected for 18 hours. The solution obtained was adjusted to pH 6.0 by adding 0.1 N NaOH, and introduced into a Sephacryl S-300 column which had previously been equilibrated with 0.1 M phosphate buffered 1 mM EDTA solution of pH 6.3, and eluted using the above phosphate buffer solution. Peak fractions corresponding to a molecular weight of about 100,000 were collected and combined and then concentrated to 1 ml to yield the desired anti-human ferritin mouse monoclonal IgG F(ab')$_2$.

### iii) Preparation of Covalently Coupled Combination and Anti-Human Ferritin Mouse Monoclonal IgG Fab'

1 ml of 0.1 M phosphate buffered 1 mM EDTA solution of pH 6.0 containing 6 mg of the above anti-human ferritin monoclonal IgG F(ab')$_2$ was mixed with 100 $\mu$l of an aqueous solution of 2-mercaptoethylamine hydrochloride (concentration of 10 mg/ml) and stirred at 37°C for 90 minutes. Gel filtration using a Sephadex G-25 column which had previously been equilibrated with 0.1 M phosphate buffer solution of pH 7.0 was carried out, and unreacted 2-mercaptoethylamine was removed. In this way, SH-Fab' was obtained. 1 mg of CHM-induced $\alpha$-amylase prepared in step i) was added, and reaction was allowed to occur at 37°C for 90 minutes. Subsequently, this reaction mixture was separated by gel filtration using a Sephacryl S-300 column which had been equilibrated with 0.1 M acetate buffered 5 mM calcium chloride solution of pH 6.0, and the fractions corresponding to molecular weights of greater than 200,000 were collected and combined. The fractions were concentrated to yield the desired covalently coupled combination.

### iv) Measurement of Ferritin in Human Serum

100 $\mu$l samples of a human serum containing human ferritin were diluted with goat serum to prepare $3^n$ dilution series. Each 100 $\mu$l sample was placed in a small test tube, and mixed with 100 $\mu$l of the combined solution prepared in the above step iii) to which were added, each in a concentration of 10 $\mu$l/ml, the amylase inhibitor produced by Streptomyces viridoporus No.297-A2 FERM-P 5405 and two kinds of anti-human ferritin monoclonal antibody whose ferritin-recognising parts have different determinants from the antibody of the combination, as well as 7% by weight of polyethylene glycol 6,000. Then, the mixture obtained was warmed at 37°C for 30 minutes. Subsequently, 1.0 ml of blue starch suspension was added to each reaction mixture, and further reaction was carried out at 37°C for 30 minutes. The enzyme reactions were terminated by adding 1 ml of 0.5 N NaOH. The mixtures were stirred, and then centrifuged at 3,500 rpm for 2 minutes. The absorbance at 620 nm of the supernatant was measured in each case and plotted against human ferritin concentration. The results are shown in Figure 10 of the accompanying drawings in which open circles indicate the readings when the two kinds of the above anti-human ferritin monoclonal antibodies were added, and the solid circles indicate the cases when these were not added. As can be seen from the figure, the sensitivity was increased to about 10 times by adding the monoclonal antibodies.

EXAMPLE 8

i) Preparation of CHM-induced α-Amylase

CHM-induced α-amylase was prepared from Bacillus subtilis α-amylase in the same manner as described in step i) of Example 4.

ii) Preparation of Anti-Human IgG Fc Specific Goat IgG F(ab')₂

Anti-human IgG goat F(ab')₂ (made by Cappel Laboratories Inc.) was passed through a Sepharose 4B bound human IgG column which had been buffered with 20 mM phosphate buffered 0.15 M NaCl solution of pH 7.0. This column was washed with the above buffer solution, and eluted using 20 mM phosphate buffered 3M NaSCN solution of pH 7.0. Subsequently, the eluate was dialysed against 0.1 M phosphate buffered 1 mM EDTA solution of pH 6.0, and concentrated to obtain the desired anti-human IgG Fc specific goat IgG F(ab')₂.

iii) Preparation of Covalently Coupled Combination of α-Amylase and Anti-Human IgG Fc Specific Goat IgG Fab'

1 ml of 0.1 M phosphate buffered 1 mM EDTA solution of pH 6.0 containing 6 mg of the above anti-human IgG Fc specific goat IgG F(ab')₂ was mixed with 100 μl of an aqueous solution of 2-mercaptoethylamine hydrochloride (concentration of 100 mg/ml) and stirred at 37°C for 90 minutes. Gel filtration was carried out using a Sephadex G-25 column which had previously been equilibrated with 0.1 M phosphate buffer solution of pH 7.0 and unreacted 2-mercaptoethylamine was removed. SH-Fab' was thus obtained. 1 mg of CHM-induced α-amylase prepared in step i) was added to the SH-Fab' solution thus obtained, and reaction was allowed to take place at 37°C for 90 minutes. Subsequently, the reaction mixture was subjected to gel filtration using a Sephacryl S-300 column which had been equilibrated with 0.1 M acetate buffered 5 mM calcium chloride solution of pH 6.0, and the fractions corresponding to molecular weights of greater than 200,000 were collected and combined. The fractions were concentrated to obtain the desired covalently coupled combination.

iv) Measurement of Human IgG

100 μl samples of human IgG solution whose concentrations were in the range of 0-1000 ng/ml were each mixed with 100 μl of the combined solution prepared in the above step iii) to which polyethylene glycol 6,000 had been added in a concentration of 7% by weight. Reaction was then allowed to occur at 37°C for 30 minutes. Subsequently, 50 μl of anti-human IgG Fab specific goat IgG (containing 10 μg of actual antibody) were added to each sample and reaction was allowed to occur at 37°C for 30 minutes. 1.0 ml of blue starch suspension was added to each reaction mixture, and further reaction took place at 37°C for 30 minutes. The enzyme reactions were terminated by adding 1 ml of 0.5 N NaOH. The mixtures were stirred, and then centrifuged at 3,500 rpm for 2 minutes. The absorbance at 620 nm of the supernatant was measured in each case and plotted on the ordinate axis against human IgG concentration as is shown in Figure 11 of the accompanying drawings. In Figure 11, open circles indicate that the anti-human IgG Fab specific goat IgG was added, and solid circles indicate that it was not added. As can be seen from the Figure, the sensitivity was raised to about 10 times by adding the anti-human IgG Fab specific goat IgG.

**Claims**

1. A method of measuring a biological ligand in a sample containing an amylase from a higher animal, which comprises bringing into contact with each other in an aqueous medium a sample derived from a higher animal containing (A) a ligand (1) to be measured and (B) the amylase (1) from the higher animal, (C)(a) an antibody (1) against said ligand (1) to which is bound an amylase (2) not contained in said sample or (b) an antibody against said ligand (1) and a biological ligand (2) to which is bound an amylase (2) not contained in said sample, said ligand (2) having at least one antigenic determinant in common with said ligand (1) and (D) an amylase inhibitor whose inhibitory activity against said amylase (1) is stronger than that against said amylase (2), said antibody reaching (a) with said ligand (1) or (b) with said ligand (1) and with said ligand (2) to which said amylase (2) is covalently coupled and said amylase inhibitor reaching with said amylase (1) digesting with said amylase (2) (E) a water insoluble macromelecular substrate of said amylase (2) and determining the residual activity of said amylase (2) and utilising the measurement thereby obtained a s a measure of said ligand (1),

and excluding the method when applied to measurement of theophylline in a sample of human serum containing amylase by bringing into contact with each other the serum sample, anti-theophylline mouse IgG as an antibody against the theophylline, a covalently coupled combination of α-amylase and theophylline and anti-human amylase goat IgG, as an inhibitor of amylase whose activity against amylase in the sample is stronger than that against α-amylase in the covalently coupled combination, said anti-theophylline mouse IgG reacting with the theophylline of the sample and with the theophylline to which the α-amylase is covalently coupled and the anti-human amylase goat IgG acting on the amylase from said sample to inhibit its activity, digesting starch with the α-amylase and determining the residual activity of the α-amylase in the covalently coupled combination and utilising the measurement obtained as a measure of the theophylline in the human serum sample.

2. The method of claim 1, wherein said amylase (2) is covalently coupled to said antibody and a further antibody (2) against an antigenic determinant of said ligand (1) which is not an antigenic determinant held in common with ligand (2) is present in said system to react with said ligand (1).

3. The method of claim 2, wherein said antibody (C) is covalently coupled to a macromolecular compound which is water-soluble and whose molecular weight is greater than 100,000 daltons.

4. The method of claim 1, wherein when said amylase (2) is covalently coupled with said ligand (2), said ligand (1) and said ligand (2) are the same substance.

5. The method of any preceding claim, wherein said ligand (1) and said ligand (2) when present is/are selected from hormones derived from endocrine glands, plasma proteins, viral antigens, bacteria, α-fetoprotein, and carcinoembryonic antigens.

6. The method of any preceding claim wherein said antibody (C) is covalently coupled with a macromolecular compound which is water-soluble and whose molecular weight is greater than 100,000 daltons.

7. The method of any preceding claim, wherein said antibody is a fragment of immunoglobulin either as such or modified by introduction of a DNP group, acetyl group, biotinyl group or nitro group.

8. The method of any one of claims 1 to 6, wherein said antibody is a monoclonal antibody.

9. The method of any preceding claim, wherein said antibody is covalently coupled to a second antibody (3).

10. The method of any preceding claim, wherein said sample is serum or urine and said amylase (1) is pancreatic amylase and or salivary amylase.

11. The method of any preceding claim, wherein said amylase (2) is α-amylase.

12. The method of any preceding claim, wherein said amylase inhibitor is selected from the amylase inhibitor derived from wheat, the amylase inhibitor produced by a microorganism of Streptomyces and an antibody against the amylase (1).

13. The method of any preceding claim, wherein said macromolecular substrate (E) is insoluble starch.

**Revendications**

EP 0 152 305 B1

1. Méthode de mesure d'un ligand biologique dans un échantillon contenant une amylase d'un animal supérieur, consistant à mettre en contact les uns avec les autres, dans un milieu aqueux, un échantillon, provenant d'un animal supérieur, qui renferme (A) un ligand (1) à mesurer et (B) l'amylase (1) de l'animal supérieur, (C) (a) un anticorps (1) contre ledit ligand (1) auquel est liée une amylase (2) non présente dans ledit échantillon, ou (b) un anticorps contre ledit ligand (1) et un ligand biologique (2) auquel est liée une amylase (2) non présente dans ledit échantillon, ledit ligand (2) ayant au moins un déterminant antigénique en commun avec ledit ligand (1), et (D) un inhibiteur d'amylase dont l'activité inhibitrice contre ladite amylase (1) est plus forte que celle contre ladite amylase (2), ledit anticorps réagissant (a) avec ledit ligand (1) ou (b) avec ledit ligand (1) et avec ledit ligand (2) auquel ladite amylase (2) est couplée de façon covalente, et ledit inhibiteur d'amylase réagissant avec ladite amylase (1), à faire digérer avec ladite amylase (2) (E) un substrat macrcomoléculaire, insoluble dans l'eau, de ladite amylase (2), et à déterminer l'activité résiduelle de ladite amylase (2) et utiliser la mesure ainsi obtenue comme mesure dudit ligand (1),
à l'exclusion de la méthode lorsqu'elle s'applique à la mesure de la théophylline dans un échantillon de sérum humain contenant une amylase, qui consiste à mettre en contact les uns avec les autres l'echantillon de sérum, une IgG de souris anti-théophylline comme anticorps contre la théophylline, une combinaison couplée par covalence d'$\alpha$-amylase et de théophylline, et une IgG de chèvre anti-amylase humaine, comme inhibiteur d'amylase dont l'activité contre l'amylase de l'échantillon est plus forte que celle contre l'amylase de la combinaison couplée par covalence, ladite IgG de souris anti-théophylline réagissant avec la théophylline de l'échantillon et avec la théophylline à laquelle l'$\alpha$-amylase est couplée par covalence et l'IgG de chèvre anti-amylase humaine agissant sur l'amylase dudit échantillon pour inhiber son activité, à faire digérer de l'amidon avec l'$\alpha$-amylase et à déterminer l'activité résiduelle de l'$\alpha$-amylase de la combinaison couplée par covalence et utiliser la mesure ainsi obtenue comme mesure de la théophylline dans l'échantillon de sérum humain.

2. Méthode de la revendication 1, dans laquelle ladite amylase (2) est couplée par covalence audit anticorps, et un autre anticorps (2) contre un déterminant antigénique dudit ligand (1), qui n'est pas un déterminant antigénique possédé en commun avec le ligand (2), est présent dans ledit système pour réagir avec ledit ligand (1).

3. Méthode de la revendication 2, dans laquelle ledit anticorps (C) est couplé par covalence avec un composé macromoléculaire soluble dans l'eau et dont le poids moléculaire est supérieur à 100000 daltons.

4. Méthode de la revendication 1, dans laquelle, lorsque ladite amylase (2) est couplée par covalence audit ligand (2), ledit ligand (1) et ledit ligand (2) sont la même substance.

5. Méthode de l'une quelconque des revendications précédentes, dans laquelle ledit ligand (1), et ledit ligand (2) lorsqu'il est présent, est/sont choisi(s) parmi les hormones provenant des glandes endocrines, les protéines plasmatiques, les antigènes viraux, les bactéries, l'$\alpha$-foetoprotéine et les antigènes carcinoembryonnaires.

6. Méthode de l'une quelconque des revendications précédentes, dans laquelle ledit anticorps (C) est couplé par covalence avec un composé macromoléculaire qui est soluble dans l'eau et dont le poids moléculaire est supérieur à 100000 daltons.

7. Méthode de l'une quelconque des revendications précédentes, dans laquelle ledit anticorps est un fragment d'immunoglobuline, tel quel ou modifié par introduction d'un groupe DNP, d'un groupe acétyle, d'un groupe biotinyle ou d'un groupe nitro.

8. Méthode de l'une quelconque des revendications 1 à 6, dans laquelle ledit anticorps est un anticorps monoclonal.

9. Méthode de l'une quelconque des revendications précédentes, dans laquelle ledit anticorps est couplé par covalence avec un second anticorps (3).

10. Méthode de l'une quelconque des revendications précédentes, dans laquelle ledit échantillon est du sérum ou de l'urine, et ladite amylase (1) est l'amylase pancréatique et/ou l'amylase salivaire.

15

**11.** Méthode de l'une quelconque des revendications précédentes, dans laquelle ladite amylase (2) est une α-amylase.

**12.** Méthode de l'une quelconque des revendications précédentes, dans laquelle ledit inhibiteur d'amylase est choisi parmi l'inhibiteur d'amylase provenant du blé, l'inhibiteur d'amylase produit par un microorganisme Streptomyces, et un anticorps contre l'amylase (1).

**13.** Méthode de l'une quelconque des revendications précédentes, dans laquelle ledit substrat macromoléculaire (E) est un amidon insoluble.

**Patentansprüche**

**1.** Verfahren zur Messung eines biologischen Liganden in einer Probe mit einem Gehalt an Amylase eines höheren Lebewesens, umfassend das miteinander Inberührungbringen in einem wässerigen Medium von einer Probe eines höheren Lebewesens enthaltend (A) einem zu messenden Liganden und (B) die Amylase des höheren Lebewesens, mit (C)(a) einem Antikörper (1) gegen den Liganden (1), an den eine nicht in der Probe enthaltene Amylase (2) gebunden ist, oder (b) einem Antikörper gegen den Liganden (1) und einen biologischen Liganden (2), an den eine nicht in der Probe enthaltene Amylase (2) gebunden ist und wobei der Ligand (2) wenigstens eine antigenische Determinante mit dem Liganden (1) gemeinsam hat, und (D) einem Amylase-Inhibitor, dessen inhibierende Wirkung gegen die Amylase (1) stärker ist als die gegen die Amylase (2), in Reaktion treten des Antikörpers (a) mit dem Liganden (1) oder (b) mit dem Liganden (1) und dem mit der Amylase (2) kovalent verbundenen Liganden (2), Einwirken des Amylase-Inhibitors auf die Amylase (1), Digerieren eines wasserunlöslichen makromolekularen Substrats (E) mit der Amylase (2), Ermittlung der restlichen Aktivität der Amylase (2) und Verwendung der so erhaltenen Messung als Maß für den biologischen Liganden,
und Ausschließen dieses Verfahrens bei Anwendung auf die Messung des Theophyllingehaltes in einer Probe von menschlichem Serum mit einem Gehalt an Amylase auch miteinander Inberührungbringen der Serumprobe, einer Antitheophyllin-IgG der Maus als Antikörper gegen Theophyllin einer kovalent verbundenen Kombination von α-Amylase und Theophyllin sowie einer antihumanen Amylase-IgG der Ziege als Amylasseinhibitor, dessen Wirkung gegen die Amylase in der Probe stärker ist als die gegen die α-Amylse in der kovalent verbundenen Kombination, wobei das Anitheophyllin-IgG der Maus reagiert wird mit dem Theophyllin in der Probe und mit dem Theophyllin, an das die α-Amylase kovalent gebunden ist, und das antihumane Amylase-IgG der Ziege auf die Amylase in der Probe unter Inhibierung der Aktivität einwirkt, Stärke mit der α-Amylase digeriert sowie die restliche Aktivität der α-Amylase in der kovalent verbundenen Mischung ermittelt wird und die so erhaltene Messung als Maß für den Theophyllingehalt im menschlichen Serum verwendet wird.

**2.** Verfahren gemäß Anspruch 1, wobei die Amylase (2) kovalent verbunden ist mit dem Antikörper und einem weiteren Antikörper (2) gegen die antigenische Determinante des Liganden (1), die keine antigenische Determinante für den Liganden (2) ist, aber im System vorliegt für die Reaktion mit dem Liganden (1).

**3.** Verfahren gemäß Anspruch 2, wobei der Antikörper (C) kovalent verbunden ist mit einer makromolekularen wasserlöslichen Verbindung deren Molekulargewicht größer als 100 000 Dalton ist.

**4.** Verfahren gemäß Anspruch 1, wobei die Amylase (2) verbunden ist mit dem Liganden (2) und der Ligand (1) und Ligand (2) dieselbe Substanz sind.

**5.** Verfahren gemäß einem jeden der vorangehenden Ansprüche, wobei der Ligand (1) und der Ligand (2), sofern er/sie anwesend ist/sind, ausgewählt wird aus Hormonen von endokrinischen Drüsen, Plasmaproteinen, Virus-Antigenen, Bakterien, α-Fetoprotein und carcinoembryonalen Antigenen.

**6.** Verfahren gemäß einem jeden der vorangehenden Ansprüche, wobei der Antikörper (C) kovalent verbunden ist mit einer wasserlöslichen makromolekularen Verbindung, deren Molekulargewicht größer als 100 000 Dalton ist.

**7.** Verfahren gemäß einem jeden der vorangehenden Ansprüche, wobei der Antikörper ein Fragment von Immunoglobulin als solchem oder in Modifikation durch Einführung einer DNP-Gruppe, Acetyl-Gruppe, Biotinyl-Gruppe oder Nitro-Gruppe ist.

8. Verfahren gemäß einem jeden der Ansprche 1 bis 6, wobei der Antikörper ein monoklonaler Antikörper ist.

9. Verfahren gemäß einem jeden der vorangehenden Ansprüche, wobei der Antikörper kovalent verbunden ist mit einem zweiten Antikörper (3).

10. Verfahren gemäß einem jeden der vorangehenden Ansprüche, wobei die Probe Serum oder Urin ist und die Amylase (1) Pankreas-Amylase oder Speichel-Amylase ist.

11. Verfahren gemäß einem jeden der vorangehenden Ansprüche, wobei die Amylase (2) $\alpha$-Amylase ist.

12. Verfahren gemäß einem jeden der vorangehenden Ansprüche, wobei der Amylase-Inhibitor ausgewählt ist aus dem von Weizen abgeleiteten Inhibitor, dem durch Stroptomyces-Mikroorganismen hergestellten Inhibitor oder einem Antikörper gegen die Amylase (1).

13. Verfahren gemäß einem jeden der vorangehenden Ansprüche, wobei das makromolekulare Substrat (E) unlösliche Stärke ist.

Fig.1.

Fig.2.

FIG.3.

FIG.4.

FIG.5.

FIG.6.

DILUTION RATIO OF SERUM

FIG.7.

FIG.8.

FIG.9.

Fig.IO.

Fig.II.